# EUROPEAN PATENT APPLICATION

(11) **EP 3 995 156 A1**
(43) Date of publication of application: **11.05.2022**
(21) Application number: 19936220.3
(22) Date of filing: 13.08.2019
(51) Int. Cl.: A61L 2/03, A61L 2/14

(54) **METHOD FOR PRODUCING A DISINFECTING SOLUTION AND DEVICE FOR CARRYING OUT SAID METHOD**

(30) Priority: 04.07.2019 RU 2019120908
(71) Applicant: Sofronov, Aleksey Vasil'evich, Nizhegorodskaya obl., 607188 (RU)
(72) Inventor: Sofronov, Aleksey Vasil'evich, Nizhegorodskaya obl., 607188 (RU)
(74) Representative: Lind, Robert
(86) International application number: PCT/RU2019/000571
(87) International publication number: WO 2021/002769

(57) **Abstract**

The claimed group of technical solutions relates to the field of disinfection of materials. In producing a disinfecting solution, moist air is fed into a discharge chamber (2) with electrodes (7, 8). Then the air passes through a charge and is saturated with active particles. To maximize the yield of long-lived radicals and increase the formation of ozone that feeds the OHH02 cycle, the air is fed into the space between an inner tube and an outer tube (6, 9) of the discharge chamber (2), and then into a piezo element chamber (5), in which the air is mixed with a finely dispersed mist from an evaporator (3), and together with the mist enters the inner tube (9) of the discharge chamber, where secondary activation of the gaseous mixture takes place. The device comprises an inner tube and an outer tube (6, 9) both made of a refractory dielectric. Moreover, the space between the inner tube (9) and the outer tube (6) is connected with the piezo element chamber (5), and the outlet of the gaseous mixture of the piezo element chamber (5) is connected with the inner tube (6).

## Description

### A method of obtaining a disinfectant solution and a device for its implementation

The field of technology.

The claimed group of technical solutions relates to the field of disinfection of materials and is intended for the rapid activation of water by low-temperature plasma of atmospheric pressure with the transformation of water into a potent disinfectant solution.

### State of the art.

It is known that water, when treated with low-temperature plasma, acquires disinfecting properties (Rohit Thimmdasa, Anjinelyulu Kothakotab, Uday Annapurec, Plasma activated water (PAW): Chemistry, physicochemical properties, applications in food and agriculture D01: 10.1016 / j.tifs.2018.05. 007, [1]). A serious disadvantage of the existing methods for treating aqueous solutions with low-temperature plasma is the long exposure time, up to several hours per ml. The disinfecting properties of water treated with plasma are associated with the formation of radicals in the OH discharge, which in turn react with nitrogen in the air and form long-lived radicals. Long exposure time is associated with a small reaction cross section for the dissociation of a water molecule by free electrons and the highest chemical reactivity of OH radicals.

Known, for example, is a method of significantly increasing the disinfecting properties of hydrogen peroxide in a plasma discharge (US patent US5785934A for invention, IPC A61L2 / 04, A61L2 / 06, A61L2 / 10, A61L2 / 14, A61L2 / 16, A61L2 / 18, A61L2 / 20, A61L2 / 24, A61L2 / 26, B01J7 / 00, B65D65 / 38, B65D65 / 40, B65D81 / 24, C01B15 / 01, C01B15 / 03, C01B15 / 037, C01B15 / 06, C01B15 / 08, C01B15 / 10, C01B15 / 14, C01B15 / 16, C01D7 / 00, 28.07.1998, [2]). As in the inventive method of vapor peroxide hydrogen enters the chamber from a hydrogen peroxide source. The articles to be sterilized are exposed to plasma by supplying energy from an RF power source to an RF electrode.

At analogue [2], a source of hydrogen peroxide is heated. In this case, the radio frequency energy used to generate the plasma can be pulsed or continuous. The devices are left in the plasma for a period to completely sterilize and / or remove residual hydrogen peroxide. In certain embodiments, 5 to 30 minutes of plasma is used.

Also known is the fluid activation system (US patent US7008592 (B2) for the invention, IPC A61L2 / 00; A61L2 / 14; A61L2 / 20; A61L2 / 22; A61L9 / 14; A61L9 / 22; B08B5 / 00; B08B5 / 02; B08B7 / 00, 07.03.2006, [3]). As in the inventive method, the starting material is fed to a generator, which forms a mist. The mist of the cleaning fluid is then activated with an AC or DC electric field, or an AC electric arc, or a DC pulsed electric field, or a DC electric arc, or an electron beam, an ion beam, or a microwave beam, or a radio frequency beam and an ultraviolet beam. generated by a laser or other source.

In analogue [3], hydrogen peroxide, peracetic acid, sodium percarbonate and glutaraldehyde are used as the starting material.

The specified analogue [3] is by the totality of essential features the closest analogue of the same purpose to the claimed method. Therefore, it is adopted as a prototype of the proposed method.

From the analogue [2], a device is known that contains a chamber with a radio frequency electrode and a source of hydrogen peroxide. The analogue [2] chamber is made with the possibility of evacuation by a pump. From the analogue [3], a device containing a fog generator and a fog activator is known. The fog machine can be designed as a low pressure ultrasonic nebulizer or a high pressure spray head. The activator can be made in the form of a pair of electric discharge plates, between which a mist of cleaning liquid passes. In analogue [3], the starting materials are hydrogen peroxide, peracetic acid, sodium percarbonate and glutaraldehyde.

Analogue [3] is adopted as a prototype of the proposed device.

A technical problem, the solution of which is provided when implementing a group of technical solutions, is the need to reactivate the gas mixture.

The disadvantage of the analogue [2] is that the energy required for the dissociation electron of the hydrogen peroxide molecule is significantly lower than water - 0.6 eV versus 8.8 eV. However, to achieve the desired effect, high concentrations of hydrogen peroxide of about 8% are required, which significantly limits the application of this method.

The disadvantage of the analogue [3] is that sterilization is carried out using OH radicals.

Disclosure of the essence of the technical solution.

The technical result provided by the claimed group of technical solutions is to maximize the yield of long-lived radicals and to increase the formation of ozone feeding the OH ↔ HO₂ cycle.

The essence of the claimed method is that moist air is supplied to the discharge chamber with electrodes. Then the air passes through the discharge and is saturated with active particles. It differs in that air is fed into the space between the outer and inner tubes of the discharge chamber, and then into the piezoelectric element chamber, in which air is mixed with fine mist from the evaporator, and enters with it into the inner tube of the discharge chamber, where the gas mixture is reactivated.

The above essence is a set of essential features of the claimed method, ensuring the achievement of the claimed technical result.

In a particular case, a high-voltage alternating voltage with an amplitude of more than 10 kV and a frequency of more than 5 kHz is supplied to the electrodes of the discharge chamber.

The essence of the claimed device lies in the fact that the device for obtaining a disinfectant solution contains a discharge chamber with electrodes and an evaporator. It differs in that the discharge chamber contains a piezoelectric element chamber, external and internal tubes made of refractory dielectric. In this case, the space between the inner tube and the outer tube communicates with the chamber of the piezoelectric element, and the outlet of the gas mixture of the chamber of the piezoelectric element is communicated with the inner tube.

The above essence is a set of essential features of the claimed device for obtaining a disinfectant solution, ensuring the achievement of the claimed technical result.

In special cases, it is permissible to perform the device as follows.

The device preferably contains a tank and a fan.

The evaporator can be ultrasonic.

The author of the claimed group of technical solutions made prototypes of these solutions, the tests of which confirmed the achievement of the technical result.

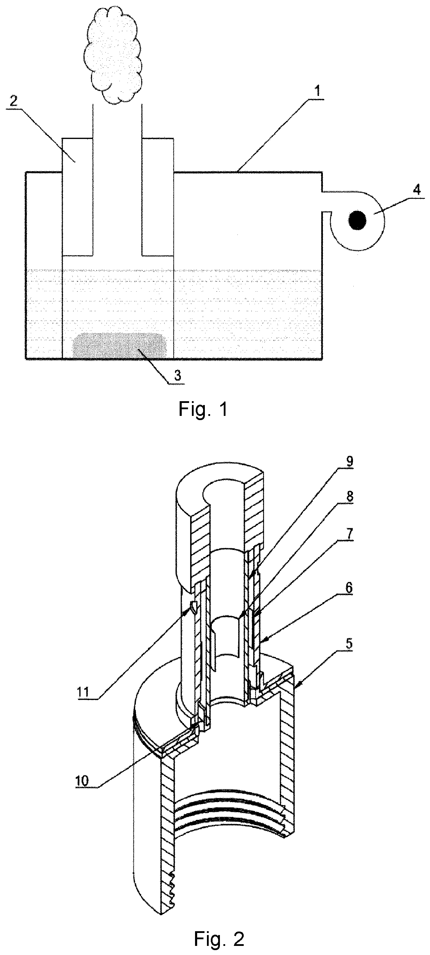

Brief description of the drawings. Figure 1 shows a general diagram of a device for obtaining a disinfectant solution; in fig. 2 is a diagram of the discharge chamber.

Implementation of a method for obtaining a disinfectant solution. The method of obtaining a disinfectant solution is as follows.

A high-voltage alternating voltage with an amplitude of more than 10 kV and a frequency of more than 5 kHz is supplied to the external and internal electrodes (7) and (8) of the discharge chamber (2) of the device for obtaining a disinfectant solution (Figs. 1, 2). A barrier dielectric discharge is ignited between the electrodes (7), (8) and the inner tube (9). Wet air is forced by a fan (4) through the holes (11) into the outer circuit of the discharge chamber (the space between the inner tube (9) and the outer tube (6)). The air passes through the discharge, while taking into account its high humidity, it is saturated with active particles. Through the holes (10), the air enters the chamber of the piezoelectric element (5) where it mixes with fine mist and together with it enters the inner tube (9) of the discharge chamber (2), where it is re-activated. An activated mist with the strongest antimicrobial properties comes out of the tube (9) of the discharge chamber (2). Mist can be used to disinfect surfaces, hands, and sterilize food.

Water activation in the proposed device becomes possible due to the interaction of free electrons with nitrogen, oxygen and water atoms, forming reactive forms of oxygen and nitrogen:

N₂ + e → 2N + e

O₂+ e → 2O + e

N + O → NO

N + O₂ → NO + O

NO + O₃ → NO₂ + O

NO₂ + N → N₂O + O

In the presence of water vapor in the plasma discharge, a small amount of nitric acid and hydrogen peroxide is synthesized:

NO₂ + O₃ → NO₃ + O₂

NO₂ + NO → N₂O₃

NO₂ + NO₃ + M → N₂O₅ + M

N₂O₅ + H₂O → 2HNO₃

N₂O₃ + H₂O → 2HNO₂

O3 + H₂O → H₂O₂ + O₂

In the above reactions, the simultaneous presence of water, nitrogen, and oxygen molecules in the discharge becomes especially important.

One of the most important active particles of plasma chemistry is the OH radical. The most likely reactions of OH formation are:
Dissipative Sticking Reaction:

   e + H₂O → H⁻ + OH
Reaction of water with oxygen ¹D:

   O + e → O(¹D) + e

   O3 + hv → O₂ + O(¹D)

   O(¹D) + H₂O → OH + OH

OH is an extremely active radical, the lifetime of which is short.

However, in the presence of ozone, the lifetime increases by several seconds due to the OH □-□ HO2 cycle:

OH + O₃ → HO₂ + O₂

HO₂ + O₃ → OH + 2O₂

In the presence of nitrous acid, hydrogen peroxide, nitric oxide and OH radicals, the synthesis of peroxynitrite and peroacetic acid can occur

HNO₂ + H₂O₂ → ONOOH + H₂O

OH^{∗} + NO₂ ↔ ONOOH

ONOO⁻ + H⁺ ↔ ONOOH

Thus, the synthesis of short-lived (OH, O) and long-lived compounds (0H ↔ OH₂, NO, NO₂, NO₃, NO₂, H₂O₂, ONOOH), which are the strongest antibacterial agents, occurs.

The implementation of the proposed method is not limited to the above example.

Implementation of a device for obtaining a disinfectant solution.

The device (Fig. 1) contains a tank (1), a discharge chamber (2), an ultrasonic evaporator (3) and a fan (4). The initial liquid in the tank can be water or a weak solution of hydrogen peroxide (0.3-0.5%).

The discharge chamber (2) consists of a piezoelement chamber (5), an outer tube of the discharge chamber made of a refractory dielectric (6), an outer electrode (7), an inner electrode (8), an inner tube of the discharge chamber made of a refractory dielectric (9), air holes the chamber of the piezoelectric element (10), openings for the air of the outer tube (11). Electrodes (7) and (8) can be stacks of conductive materials, spirals of conductive wire, tubes of conductive material, or any other conductors of a cellular structure.

Examples of specific implementation of the device.

Example 1. The initial solution for the evaporator (3) of the device is a weak solution of hydrogen peroxide up to 1%.

Example 2. The initial solution for the evaporator (3) of the device is a weak alcohol solution up to 10%.

Description of work and order of using the device.

To the outer and inner electrodes (7), (8) of the discharge chamber (2)

(Fig. 1, 2) serves a high-voltage alternating voltage with an amplitude of more than 10 kV and a frequency of more than 5 kHz. Between electrodes (7), (8) and the inner tube (9) ignites a barrier dielectric discharge. Wet air is forced by a fan (4) through the holes (11) into the outer circuit of the discharge chamber (the space between the inner tube (9) and the outer tube (6)). The air passes through the discharge, while taking into account its high humidity, it is saturated with active particles. Through the holes (10), the air enters the chamber of the piezoelectric element (5) where it mixes with fine mist and together with it enters the inner tube (9) of the discharge chamber (2), where it is re-activated. An activated fog with antimicrobial properties comes out of the tube (9) of the discharge chamber (2).

### Industrial applicability.

The claimed group of technical solutions is implemented using commercially available devices and materials and can be applied at any enterprise where disinfection of materials is required.

## Claims

1. A method of obtaining a disinfectant solution, including supplying moist air to the discharge chamber with electrodes, passing air through the discharge and saturating the air with active particles, **characterized in that** air is supplied into the space between the outer and inner tubes of the discharge chamber, and then into the piezoelectric cell, in which air mixes with fine mist from the evaporator, and together with the mist enters the inner tube of the discharge chamber, where the gas mixture is reactivated.

2. The method according to claim 1, **characterized in that** a high-voltage alternating voltage with an amplitude of more than 10 kV and a frequency of more than 5 kHz is supplied to the electrodes of the discharge chamber.

3. A device for producing a disinfectant solution containing a discharge chamber with electrodes and an evaporator, **characterized in that** the discharge chamber contains a piezoelectric cell, an outer and an inner tube made of a refractory dielectric, while the space between the inner tube and the outer tube is communicated with the piezoelectric cell, and the gas mixture outlet the chamber of the piezoelectric element is in communication with the inner tube.

4. The device according to claim 3, **characterized in that** it contains a tank and a fan.

5. The device according to claim 3, **characterized in that** the evaporator is ultrasonic.
